# EUROPEAN PATENT APPLICATION

(11) **EP 4 606 906 A1**
(43) Date of publication of application: **27.08.2025**
(21) Application number: 25156630.3
(22) Date of filing: 07.02.2025
(51) Int. Cl.: C12Q 1/26, C12Q 1/28, C12Q 1/54

(54) **KIT FOR QUANTITATIVE ANALYSIS OF 1,5-ANHYDROGLUCITOL, METHOD FOR QUANTITATIVE ANALYSIS OF 1,5-ANHYDROGLUCITOL, AND APPARATUS FOR PERFORMING THE SAME**

(30) Priority: 23.02.2024 KR 20240026230
(71) Applicant: I-SENS, INC., Seoul 06646 (KR)
(72) Inventor: Kim, Ji Hoon, 06646 Seoul (KR); Park, Ji Yeon, 06646 Seoul (KR); Kim, Jin Young, 06646 Seoul (KR); Kim, Seung Wan, 06646 Seoul (KR); Choi, Dong Cheol, 06646 Seoul (KR); Kim, Hyeong Eun, 06646 Seoul (KR)
(74) Representative: Westphal, Mussgnug & Partner Patentanwälte mbB

(57) **Abstract**

The kit for quantitative analysis of 1,5-anhydroglucitol (1,5-AG) according to an embodiment of the present application may comprise: a first composition comprising a glucose removal reagent for removing at least a portion of glucose in a sample; a second composition comprising a glucitol reaction reagent for enzyme reaction of 1,5-anhydroglucitol included in the sample from which at least a portion of glucose has been removed; and a third composition comprising a chromogenic agent being oxidized from hydrogen peroxide (H2O2) generated by enzyme reaction of the 1,5-anhydroglucitol.

## Description

### CROSS-REFERENCE TO RELATED APPLICATION

This application claims priority to and the benefit of Korean Patent Application No. 10-2024-0026230, filed on February 23, 2024, the disclosure of which is incorporated herein by reference in its entirety.

### BACKGROUND

### 1. Field of the Invention

The present application relates to quantitative analysis of biological samples, and more specifically to a kit for quantitative analysis of 1,5-anhydroglucitol (1,5-AG), a method for quantitative analysis of 1,5-AG, and/or a device for performing the same.

### 2. Discussion of Related Art

In diagnosing diabetes, along with blood glucose measurement, the need for testing 1,5-anhydroglucitol (hereinafter referred to as 1,5-AG) is increasing. 1,5-AG is a type of glucose derivative known to be chemically and biochemically stable, maintaining a constant blood concentration. In particular, 1,5-AG has a structure similar to glucose and is characterized by competitive reabsorption with glucose. That is, when blood glucose concentration increases, 1,5-AG concentration decreases, and when blood glucose concentration decreases, 1,5-AG concentration increases. Technical development and research utilizing this correlation between blood glucose and 1,5-AG for diagnosing diabetes through quantitative analysis of 1,5-AG are receiving attention.

The conventional technology for 1,5-AG quantitative analysis used a combination of two reagents as chromogenic agents: either 4-Aminoantipyrine (4-AAP) with Hydroxytoluenesulfonyliodobenzene (HTIB), or 4-AAP with N-Ethyl-N-(2-hydroxy-3-sulfopropyl)-3-methylaniline sodium salt (TOOS). Due to the separate configuration of these two reagents, there were limitations in miniaturizing the quantitative analysis kit and/or device, resulting in 1,5-AG analysis being typically implemented on large biochemical equipment. Furthermore, the conventional chromogenic reagents had limitations in their coloring ability under low-concentration hydrogen peroxide conditions.

Therefore, there is a need for technical development and research on 1,5-AG quantitative analysis that can be miniaturized and has improved analytical capabilities.

### SUMMARY OF THE INVENTION

One objective of the present invention is to provide a kit for quantitative analysis of 1,5-anhydroglucitol, a method for quantitative analysis of 1,5-anhydroglucitol, and/or a device for performing the same, that can be miniaturized.

Another objective of the present invention is to provide a kit for quantitative analysis of 1,5-anhydroglucitol, a method for quantitative analysis of 1,5-anhydroglucitol, and/or a device for performing the same, comprising a composition for enhancing analytical capability of 1,5-AG

Objects of the present invention are not limited to the above-described objects and other objects that are not described may be clearly understood by those skilled in the art from this specification and the accompanying drawings.

The kit for quantitative analysis of 1,5-anhydroglucitol (1,5-AG) according to an embodiment of the present application may comprise: a first composition comprising a glucose removal reagent for removing at least a portion of glucose in a sample; a second composition comprising a glucitol reaction reagent for enzyme reaction of 1,5-anhydroglucitol included in the sample from which at least a portion of glucose has been removed; and a third composition comprising a chromogenic agent being oxidized from hydrogen peroxide (H2O2) generated by enzyme reaction of the 1,5-anhydroglucitol, wherein the kit for quantitative analysis of 1,5-AG may comprise at least one reagent fixing part, wherein the second composition and the third composition may be independently fixed in solid state to the reagent fixing part in respectively, wherein the second composition may further comprise a stabilizer for fixing the glucitol reaction reagent in solid state to the reagent fixing part, and wherein the chromogenic agent may be DA-67.

Solutions of the present invention are not limited to the above-described solutions and other solutions that are not described may be clearly understood by those skilled in the art from this specification and the accompanying drawings.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a schematic diagram of a kit for quantitative analysis of 1,5-anhydroglucitol (1,5-AG) according to an embodiment of the present application.
FIG. 2 is a schematic diagram of a sample collector of the 1,5-AG quantitative analysis kit according to an embodiment of the present application.
FIG. 3 is a schematic diagram of the main cartridge of the 1,5-AG quantitative analysis kit according to an embodiment of the present application.
FIG. 4 is a schematic diagram of a solution cell of the 1,5-AG quantitative analysis kit according to an embodiment of the present application.
FIG. 5 is a diagram illustrating the aspects where the reaction buffer stored in the solution cell flows into the mixing region of the main cartridge as the sample collector is inserted into the main cartridge, according to an embodiment of the present application.
FIG. 6 is a diagram illustrating the aspect of reaction mechanism used for quantitative analysis according to an embodiment of the present application.
FIGs. 7 and 8 are diagrams illustrating the detailed structure of the 1,5-AG quantitative analysis kit according to an embodiment of the present application.
FIG. 9 is a diagram illustrating the analysis aspect for quantifying 1,5-AG in a sample according to an embodiment of the present application.
FIG. 10 is a graph and table showing evaluation results of absorbance by chromogenic agent according to one embodiment of the present application.
FIG. 11 is a table and graph showing evaluation results of stability according to state of the glucitol reaction reagent according to one embodiment of the present application.
FIG. 12 is a graph showing evaluation results of stability according to state of the glucitol reaction reagent according to one embodiment of the present application.
FIG. 13 is a graph showing evaluation results of absorbance according to stabilizer added to the glucitol reaction reagent according to one embodiment of the present application.
FIG. 14 is a table showing evaluation results of absorbance according to concentration of stabilizer added to the glucitol reaction reagent according to one embodiment of the present application.
FIG. 15 is a graph showing evaluation results of absorbance according to concentration of stabilizer added to the glucitol reaction reagent according to one embodiment of the present application.
FIG. 16 is a diagram showing identification information included in the 1,5-AG quantitative analysis kit according to an embodiment of the present application.

### DETAILED DESCRIPTION OF EXEMPLARY EMBODIMENTS

The above-described objects, features and, advantages of the present invention will be clearly understood through the following detailed description taken in conjunction with the accompanying drawings. However, while the present invention can be modified in various ways and take on various alternative forms, specific embodiments thereof are shown in the drawings and described in detail below as examples.

Like reference numerals refer to like elements in principle throughout the specification. Further, elements with the same function within the scope of the same idea shown in the drawings of each embodiment will be described using the same reference numerals, and the descriptions thereof will not be repeated.

When it is determined that detailed descriptions of related well-known functions or configurations may unnecessarily obscure the gist of the present invention, detailed descriptions thereof will be omitted. Further, the ordinal numbers (for example, first, second, etc.) used in description of the specification are used only to distinguish one element from another element.

Further, the term "module," "unit," "part," or "portion" of an element used herein is assigned or incorporated for convenience of specification description, and the term itself does not have a distinct meaning or role.

As used herein, the singular forms "a" and "an" are intended to include the plural forms as well, unless the context clearly indicates otherwise.

It will be further understood that the terms "comprise," "comprising," "include," and/or "including" used herein specify the presence of stated features or elements, but do not preclude the presence or addition of one or more other features or elements.

Sizes of elements in the drawings may be exaggerated for convenience of explanation. In other words, since sizes and thicknesses of elements in the drawings are arbitrarily illustrated for convenience of explanation, the following embodiments are not limited thereto.

When a certain embodiment may be implemented differently, a specific process order may be performed differently from the described order. For example, two consecutively described processes may be performed substantially at the same time or performed in an order opposite to the described order.

In the following embodiments, when a first element is referred to as being "connected" to a second element, it includes not only a case where the two elements are "directly connected," but also a case where the two elements are "indirectly connected" with a third element interposed therebetween. For example, in this specification, when a first element is referred to as being "electrically connected" to a second element, it includes not only a case where the two elements are "directly electrically connected," but also a case where the two elements are "indirectly electrically connected" with a third element interposed therebetween.

The kit for quantitative analysis of 1,5-Anhydroglucitol (1,5-AG) according to an embodiment of the present application may comprise: a first composition comprising a glucose removal reagent for removing at least a portion of glucose in a sample; a second composition comprising a glucitol reaction reagent for enzyme reaction of 1,5-anhydroglucitol included in the sample from which at least a portion of glucose has been removed; and a third composition comprising a chromogenic agent being oxidized from hydrogen peroxide (H2O2) generated by enzyme reaction of the 1,5-anhydroglucitol, wherein the kit for quantitative analysis of 1,5-AG may comprise at least one reagent fixing part, wherein the second composition and the third composition may be independently fixed in solid state to the reagent fixing part in respectively, wherein the second composition may further comprise a stabilizer for fixing the glucitol reaction reagent in solid state to the reagent fixing part, and wherein the chromogenic agent may be DA-67.

According to an embodiment of the present application, wherein the stabilizer may be selected from the group consisting of sucrose, trehalose, lactose, maltose, turanose, cellobiose, dextran, diethylamino ethyl-dextran, dextrin, cellulose, and beta-glucans.

According to an embodiment of the present application, wherein the stabilizer may be sucrose, and wherein the sucrose may be added to the second composition at a concentration within a range of 90 mM to 360 mM.

According to an embodiment of the present application, wherein the stabilizer may be sucrose, and wherein the sucrose may be added to the second composition at a concentration within a range of 160 mM to 200 mM.

According to an embodiment of the present application, wherein the glucose removal reagent may comprise: a glucose converting enzyme for generating a product including glucose-6-phosphate from a reactant including the glucose in the sample; and a reverse reaction removal reagent for inhibiting a reverse reaction from the glucose-6-phosphate back to the glucose.

According to an embodiment of the present application, wherein the glucose converting enzyme may be selected from the group consisting of glucokinase (GK), hexokinase, and mixtures thereof.

According to an embodiment of the present application, wherein the reverse reaction removal reagent may comprise pyruvate kinase and phosphoenolpyruvic acid (PEP).

According to an embodiment of the present application, wherein the reactant may further comprise ATP.

According to an embodiment of the present application, wherein the glucitol reaction reagent may comprise: a first reaction enzyme generating hydrogen peroxide by reacting with the 1,5-AG in the sample; and a second reaction enzyme oxidizing the chromogenic agent by reacting with the hydrogen peroxide.

According to an embodiment of the present application, wherein the first reaction enzyme may be pyranose oxidase (PROD).

According to an embodiment of the present application, wherein the second reaction enzyme may be peroxidase (POD).

According to an embodiment of the present application, wherein by fixing and storing the second composition comprising the glucitol reaction reagent in solid state to the reagent fixing part, the activity of the glucitol reaction reagent may be maintained at 66% or more of its initial activity even after 38 days from the start of storage.

According to an embodiment of the present application, wherein by fixing and storing the second composition comprising the glucitol reaction reagent in solid state to the reagent fixing part, the activity of the glucitol reaction reagent may be maintained at 85% or more of its initial activity even after 38 days from the start of storage.

According to an embodiment of the present application, wherein by fixing and storing the second composition comprising the glucitol reaction reagent in solid state to the reagent fixing part, the activity of the glucitol reaction reagent may be maintained at 66% or more of its initial activity even after 326 days from the start of storage.

According to an embodiment of the present application, wherein by fixing and storing the second composition comprising the glucitol reaction reagent in solid state to the reagent fixing part, the activity of the glucitol reaction reagent may be maintained at 80% or more of its initial activity even after 326 days from the start of storage.

According to an embodiment of the present application, wherein the kit may comprise a body including an upper plate and a lower plate configured to face each other, wherein the second composition may be fixed to a first reagent fixing part located in a first compartmental region of the upper plate, wherein the third composition may be fixed to a second reagent fixing part located in the first compartmental region of the lower plate, and wherein the first reagent fixing part and the second reagent fixing part may be configured to face each other.

According to an embodiment of the present application, wherein the kit may comprise a solution cell disposed in a second compartmental region partitioned from the first compartmental region, wherein the first composition may be stored in the solution cell, wherein when a sample collector containing the sample is inserted into a receiving part of the kit, the first composition contained in the solution cell and the sample contained in the sample collector may be configured to flow into a mixing region within the second compartmental region such that at least a portion of glucose in the sample may be removed in the mixing region.

According to an embodiment of the present application, wherein the kit may further comprise a flow path connecting the first compartmental region and the second compartmental region, wherein the sample from which at least a portion of glucose has been removed may flow from the mixing region within the second compartmental region to the first compartmental region through the flow path as the kit is rotated by a predetermined angle by external force applied to the kit.

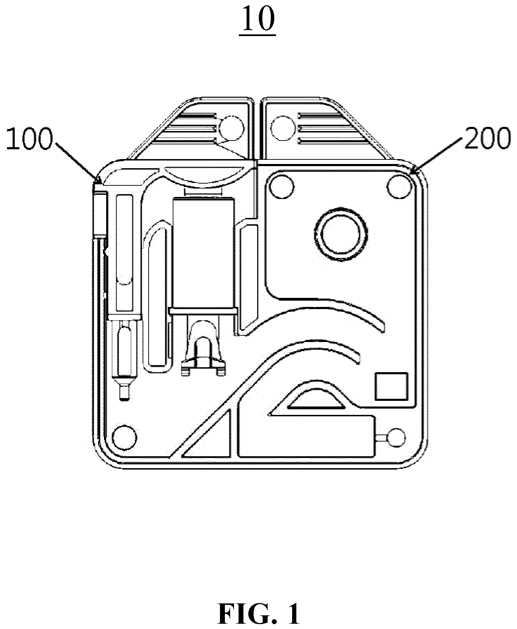

Hereinafter, with reference to Figures 1 to 16, the structure of the kit for quantitative analysis of 1,5-Anhydroglucitol (1,5-AG), the method for quantitative analysis of 1,5-AG, and/or the apparatus for quantitative analysis of 1,5-AG according to an embodiment of the present application will be described in greater detail.

FIG. 1 is a schematic diagram of a kit for quantitative analysis of 1,5-Anhydroglucitol (1,5-AG) according to an embodiment of the present application.

The kit for quantitative analysis of 1,5-Anhydroglucitol (hereinafter referred to as "1,5-AG") 10 according to an embodiment of the present application may include a sample collector 100 for supplying a biological sample and/or a main cartridge 200 into which the sample collector 100 can be inserted and received.

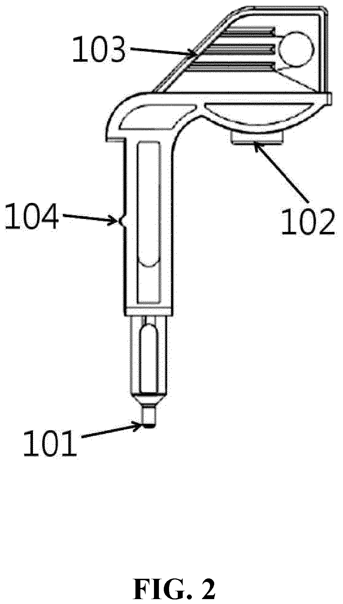

FIG. 2 is a schematic diagram of a sample collector 100 of the 1,5-AG quantitative analysis kit according to an embodiment of the present application.

The sample collector 100 according to an embodiment of the present application may be configured to collect a predetermined amount of a biological sample (e.g., a serum sample, and/or a plasma sample) intended for analysis. Specifically, the sample collector 100 may include a capillary-type sample inlet 101 and may collect the biological sample to be analyzed through the capillary provided in the sample inlet 101, injecting the collected biological sample into the main cartridge 200.

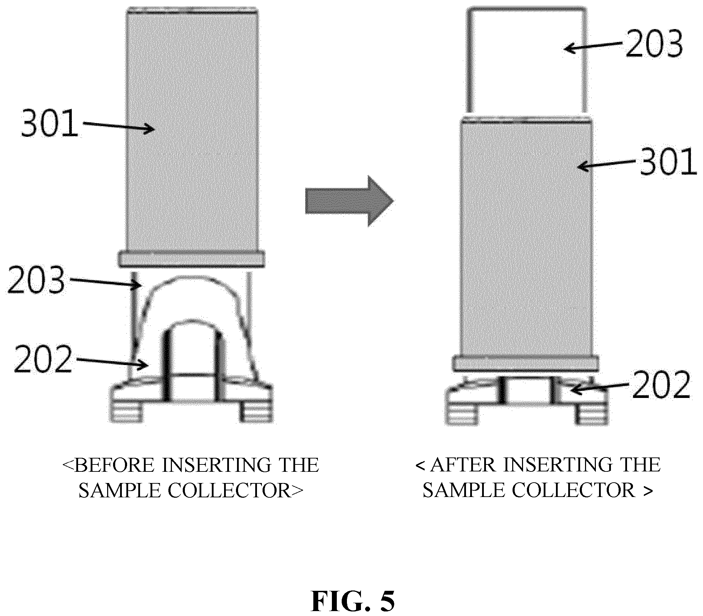

Furthermore, the sample collector 100 may include a protrusion 102 designed to contact a solution cell 301 inside the main cartridge 200 and push it inward when the sample collector 100 is inserted into the receiving part 201 of the main cartridge 200, described later. Specifically, the protrusion 102 may be positioned on the sample collector 100 to contact the solution cell 301 when the sample collector 100 is inserted into the receiving part 201 of the main cartridge 200. Consequently, as the sample collector 100 is inserted into the receiving part 201 of the main cartridge 200, the solution cell 301 may be moved inward toward the interior of the main cartridge 200, causing the cover tape of the solution cell 301 to be removed or ruptured. This will be described in more detail with reference to Fig. 5.

Additionally, the sample collector 100 may further include a handle 103 and/or a locking clip 104. Specifically, the handle 103 is a component designed to facilitate the transportation or use of the sample collector 100 and is not limited to the structure shown in Fig. 2. The locking clip 104 may be provided on one side of the sample collector 100 to secure the sample collector 100 to the main cartridge 200 when inserted into main cartridge 200. The locking clip 104 is designed to match the size and shape of a locking groove 211 provided in the receiving part 201 of the main cartridge 200. The locking clip 104 of the sample collector 100 and the locking groove 211 of the main cartridge 200 can engage with each other. As a result, the inserted sample collector 100 may be fixed to the main cartridge 200, preventing movement or detachment of the sample collector 100 even if the 1,5-AG quantitative analysis kit rotates during analysis.

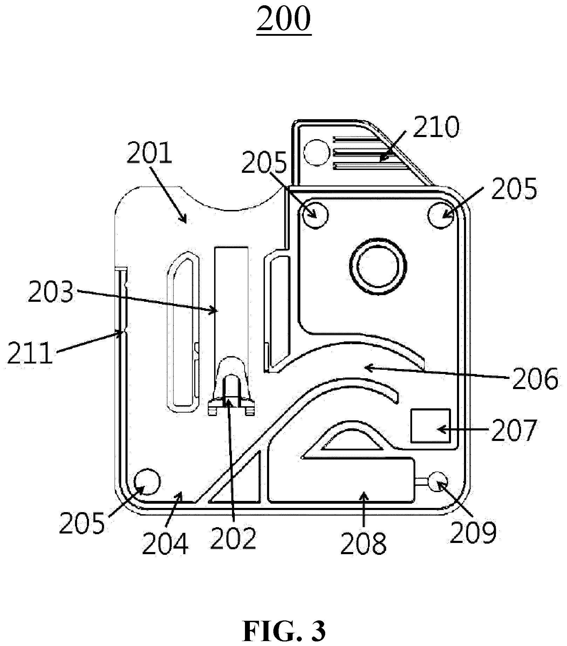

FIG. 3 is a schematic diagram of the main cartridge 200 of the 1,5-AG quantitative analysis kit according to an embodiment of the present application.

The main cartridge 200 according to an embodiment of the present application may include a receiving part 201 into which the sample collector 100 can be inserted. Furthermore, the receiving part 201 may include a locking groove 211 configured to engage with the locking clip 104 of the sample collector 100, as described above, to secure the sample collector 100.

The main cartridge 200 may also include a moving frame 203 configured to fix and move the solution cell 301 provided inside the main cartridge. Specifically, the moving frame 203 may be configured to secure the solution cell 301 prior to the insertion of the sample collector 100. When the sample collector 100 is inserted, the solution cell 301, pushed by the protrusion 102 of the sample collector 100, may move along a moving path in the moving frame 203.

The main cartridge 200 may further include a cover tape rupturing part 202 configured to remove or rupture the cover tape 302 of the solution cell 301 as the solution cell 301 moves along the moving frame 203. This will be described in greater detail with reference to Fig. 5.

The main cartridge 200 may include a mixing part 204 (or mixing region) where the biological sample discharged from the sample collector 100 through the receiving part 201 mixes with the composition (Hereinafter referred to as a first composition) discharged from the solution cell 301.

The main cartridge 200 may also include at least one reagent fixing part 205 (referred to as a sample fixing part) introduced with chemical reagents that can react with the first composition and biological sample mixed in the mixing part 204 to induce enzymatic reaction and/or antigen-antibody reactions.

The main cartridge 200 may further include a flow path 206 through which the mixed first composition and biological sample in the mixing part 204 can move. Specifically, the analysis sample may travel, through the flow path 206, between the mixing part 204, the reagent fixing part 205, and/or a measurement unit 207 that optically measures the analysis sample. The structure of the flow path 206 is not limited, provided it is designed to allow the analysis sample to move by gravity when the main cartridge 200 is tilted.

The main cartridge 200 according to an embodiment of the present application may include a measurement unit 207 for measuring the reaction results performed at the reagent fixing part 205. The 1,5-AG quantitative analysis apparatus according to an embodiment of the present application can quantify the analysis sample (e.g., 1,5-AG) through optical analysis, such as UV/VIS, via the measurement unit 207.

The main cartridge 200 may include a waste solution treatment part 208 for collecting waste solutions which analysis was completed via the measurement unit 207. The waste solution treatment part 208 allows for the collection and separate disposal of waste solutions, which are a type of medical waste. Collection of waste solutions via the waste solution treatment part 208 can be achieved by absorbing the waste solution into highly absorbent cotton, absorbent filters, or polymer-based absorbent materials, placed in the waste solution treatment part 208.

The main cartridge 200 may further include an air vent 209 to facilitate the smooth movement and absorption of the waste solution into the absorbent materials. Through the air vent 209, the transfer of waste solutions to the waste solution treatment part 208 and their subsequent collection can be more efficiently performed.

The main cartridge 200 may also include a handle 210 to facilitate its transportation and use. The structure of the handle 210 is not limited to the form shown in Fig. 3.

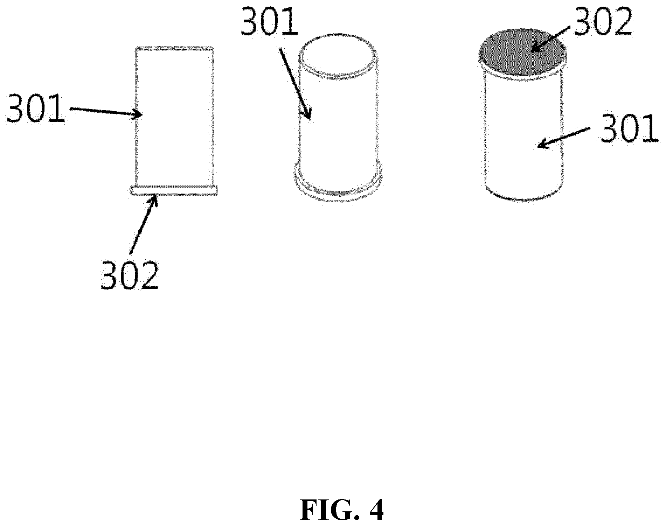

Hereinafter, with reference to Figures 4 and 5, a more detailed description will be provided of the aspects in which the first composition of the solution cell 301 flows into the mixing part 204 of the main cartridge 200 when the sample collector 100 is inserted into the receiving part 201 of the main cartridge 200. FIG. 4 is a schematic diagram of a solution cell 301 of the 1,5-AG quantitative analysis kit according to an embodiment of the present application. FIG. 5 is a diagram illustrating the aspects where the first composition stored in the solution cell 301 flows into the mixing region of the main cartridge 200 as the sample collector 100 is inserted into the main cartridge 200, according to an embodiment of the present application.

The main cartridge 200 according to an embodiment of the present application may include a solution cell 301 that stores a first composition for reacting with the biological sample from the sample collector 100. The solution cell 301 includes an opening at one end, sealed with a cover tape 302, to prevent the first composition stored inside from leaking. The opening sealed with the cover tape 302 of solution cell 301 is positioned to face the cover tape rupturing part 202 of the main cartridge 200.

Before the sample collector 100 is inserted into the receiving part 201 of the main cartridge 200, the opening of the solution cell 301 sealed with the cover tape 302 may be positioned apart from the cover tape rupturing part 202. Before the sample collector 100 is inserted into the receiving part 201 of the main cartridge 200, the opening of the solution cell 301 sealed with the cover tape 302 may be positioned apart from the cover tape rupturing part 202. When the sample collector 100 is inserted into the receiving part 201 of the main cartridge 200, the protrusion 102 of the sample collector 100 applies pressure to the opposite end of the solution cell 301, away from the opening sealed with the cover tape 302. This applied pressure causes the solution cell 301 to move along the moving path of the moving frame 203.

As a result, when the cover tape 302 of the solution cell 301 comes into contact with the cover tape rupturing part 202, the cover tape 302 of the solution cell 301 is removed or ruptured. The first composition stored in the solution cell 301 is then transferred through a flow path or hollow structure formed in the cover tape rupturing part 202 into the mixing part 204 of the main cartridge 200. In the mixing part 204, the reaction buffer mixes with the biological sample supplied from the sample collector 100.

Specifically, the first composition flowing into the mixing part 204 may be designed to contact the sample inlet 101 of the sample collector 100. This contact allows the biological sample contained in the capillary-type sample inlet 101 to move through the sample inlet 101 into the mixing part 204 of the main cartridge 200. Consequently, in the mixing part 204 of the main cartridge 200, the first composition stored in the solution cell 301 and the biological sample collected by the sample collector 100 can be mixed.

In Figures 4 and 5, the shape of the solution cell 301 is illustrated as a specific example. However, this is merely an example, and the shape of the solution cell 301 is not limited as long as it provides a structure suitable for storing the first composition. Similarly, the material of the cover tape 302 is not particularly limited as long as it prevents the first composition from leaking while allowing for easy removal or rupture.

Additionally, Figure 5 illustrates the cover tape rupturing part 202 in a specific shape for explanation purposes. However, this is also merely an example, and the shape of the cover tape rupturing part 202 is not limited, provided it facilitates the removal or rupture of the cover tape. Suitable structures include, but are not limited to, needle-shaped or edge-shaped configurations.

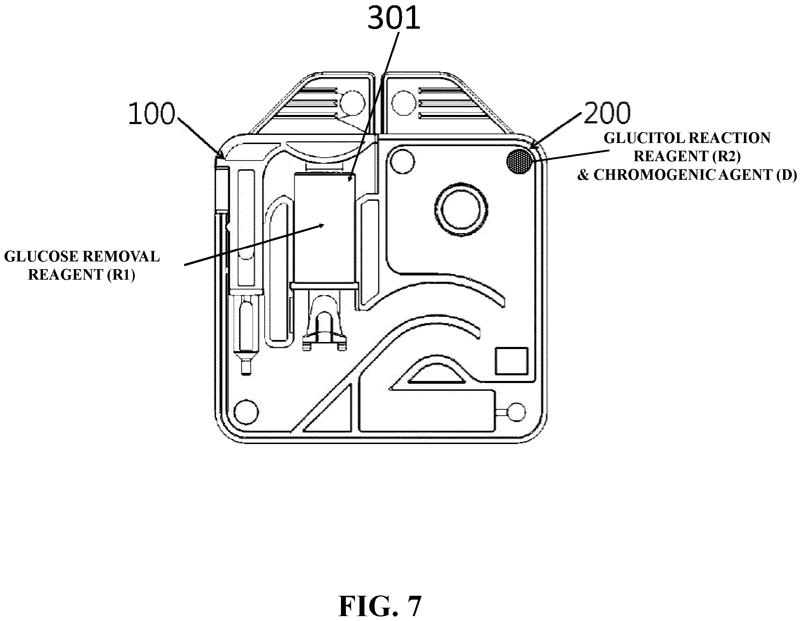

The 1,5-AG quantitative analysis kit 10 according to an embodiment of the present application may be used to quantitatively analyze 1,5-AG present in blood (e.g., plasma, serum sample). According to one embodiment, the 1,5-AG quantitative analysis kit 10 may be configured to quantitatively analyze 1,5-AG using an enzymatic method. Hereinafter, with reference to Figures 7 to 9, a more detailed description will be provided of the aspects of quantitatively analyzing 1,5-AG using the 1,5-AG quantitative analysis kit 10 according to an embodiment of the present application.

The enzymatic method for quantifying 1,5-AG consists of two main chemical reactions.

The first reaction is for removing at least a portion of glucose included in the sample, as glucose competitively reacts with the reaction enzyme that reacts with 1,5-AG due to their similar structures. Therefore, before the main reaction of 1,5-AG, a pre-treatment reaction to remove glucose included in the sample is required as the first reaction.

According to one embodiment of the present application, the method for quantitative analysis of 1,5-AG may use a reaction of converting glucose included in the sample to glucose-6-phosphate using glucose removal reagent as the first reaction. Meanwhile, to prevent the reverse reaction of converting glucose to glucose-6-phosphate, namely the reaction of glucose-6-phosphate returning to glucose, the reaction of converting glucose to glucose-6-phosphate may be coupled with a reaction of converting phosphoenolpyruvic acid (PEP) to pyruvate.

The second reaction is a color development reaction using hydrogen peroxide (H2O2) generated in proportion to the concentration of 1,5-AG included in the sample. According to one embodiment of the present application, the method for quantitative analysis of 1,5-AG may use, as the second reaction, a reaction of generating hydrogen peroxide from 1,5-AG using glucitol reaction reagent, oxidizing the generated hydrogen peroxide, and reducing of the chromogenic agent and changing color of the chromogenic agent, due to electrons released by oxidation.

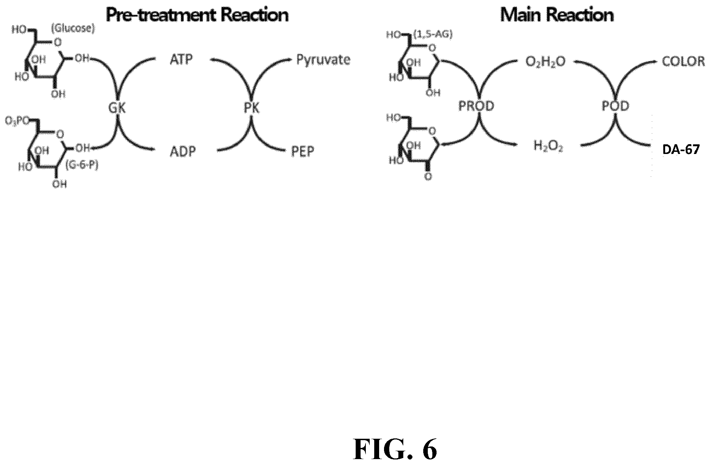

According to one embodiment of the present application, 1,5-AG may be quantitatively analyzed using the reaction mechanism shown in FIG. 6. FIG. 6 is a diagram illustrating the aspect of reaction mechanism used for quantitative analysis according to an embodiment of the present application.

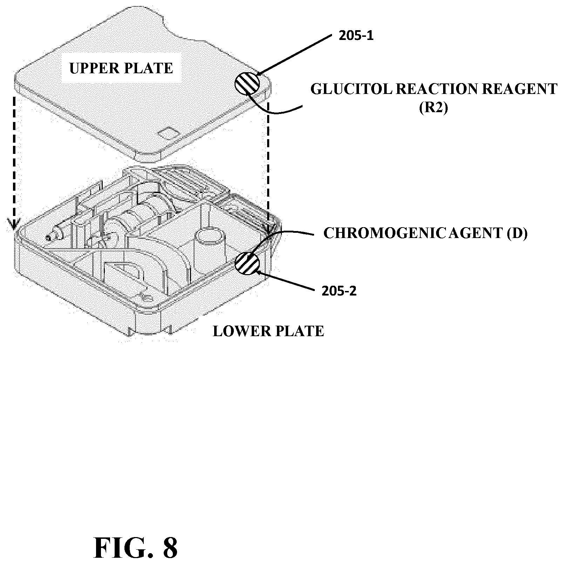

FIGs. 7 and 8 are diagrams illustrating the detailed structure of the 1,5-AG quantitative analysis kit 10 according to an embodiment of the present application.

The solution cell 301 of the kit for quantitative analysis of 1,5-AG 10 according to one embodiment of the present application may store a first composition comprising a glucose removal reagent R1 for inducing a reaction to remove at least a portion of glucose included in the sample. As described above, when the sample collector 100 is inserted, the glucose removal reagent R1 stored in the solution cell 301 moves to the mixing part 204 of the main cartridge 200. At this time, the glucose removal reagent R1 is mixed with the sample collected through the sample collector 100 in the mixing part 204. During the mixing process, at least a portion of glucose included in the sample can be removed through the glucose removal reagent R1.

According to one embodiment of the present application, the glucose removal reagent R1 may include a glucose converting enzyme for converting glucose in the sample to glucose-6-phosphate and/or a reverse reaction removal reagent for removing the reverse reaction from glucose-6-phosphate back to glucose.

According to one embodiment, the glucose converting enzyme may be selected from the group consisting of glucokinase (GK), hexokinase, and mixtures thereof. In a preferred embodiment, the glucose converting enzyme may be glucokinase (GK).

According to one embodiment, the reverse reaction removal reagent may include pyruvate kinase and phosphoenolpyruvic acid (PEP). Furthermore, the glucose removal reagent R1 may further include ATP as a reactant for coupling between the glucose conversion reaction and reverse reaction. However, this is merely exemplary, and any suitable composition that can remove glucose without affecting the quantitative analysis of 1,5-AG in the sample may be used. For example, the reverse reaction removal reagent may include acetate kinase and acetyl phosphate. For example, the reverse reaction removal reagent may include creatine kinase and creatine phosphate.

The sample from which at least a portion of glucose has been removed by the glucose removal reagent R1 can move through the flow path 206 to the measurement unit 207 and/or at least one reagent fixing part 205. At least one reagent fixing part 205 may have fixed thereon a composition (Hereinafter, a second composition) comprising a glucitol reaction reagent R2 for enzyme reaction of 1,5-AG present in the glucose-removed sample and/or a composition (Hereinafter, a third composition) comprising a chromogenic agent D oxidized from hydrogen peroxide generated by enzyme reaction of 1,5-AG

According to one embodiment, the glucitol reaction reagent R2 may include a first reaction enzyme generating hydrogen peroxide by reacting with 1,5-AG contained in the sample from which at least a portion of glucose has been removed, and a second reaction enzyme oxidizing the chromogenic agent by reacting with the hydrogen peroxide. According to one embodiment, the first reaction enzyme may be pyranose oxidase (PROD). According to one embodiment, the second reaction enzyme may be peroxidase (POD).

According to one embodiment, the chromogenic agent D may be Sodium 10-(carboxymethylaminocarbonyl)-3,7-bis(dimethylamino)phenothiazine (hereinafter, DA-67). According to one embodiment, the kit for quantitative analysis of 1,5-AG, method for quantitative analysis of 1,5-AG, and/or device for quantitative analysis of 1,5-AG can provide an effect of more precisely differentiating hydrogen peroxide in low concentration ranges by using DA-67 as the chromogenic agent.

According to one embodiment, the second composition comprising glucitol reaction reagent R2 and the third composition comprising chromogenic agent D may be independently fixed on at least one reagent fixing part 205 in respectively. Referring to FIG. 8, the kit 10 for quantitative analysis of 1,5-AG may include a body comprising an upper plate and a lower plate configured to face each other. At this time, the second composition comprising glucitol reaction reagent R2 may be fixed to a first reagent fixing part 205-1 located on the upper plate of the kit 10 for quantitative analysis of 1,5-AG Meanwhile, the third composition comprising chromogenic agent D may be fixed to a second reagent fixing part 205-2 located on the lower plate of the kit 10 for quantitative analysis of 1,5-AG. Furthermore, the first reagent fixing part 205-1 and second reagent fixing part 205-2 may be located in a first compartmental region of the kit 10 for quantitative analysis of 1,5-AG in a facing configuration. Meanwhile, the first compartmental region where the first reagent fixing part 205-1 and second reagent fixing part 205-2 are located may be partitioned from a second compartmental region where the mixing region is located where the first composition from the solution cell and the sample from the sample collector are mixed, and the first compartmental region and second compartmental region may be connected through a flow path.

According to one embodiment, the second composition comprising glucitol reaction reagent R2 and/or the third composition comprising chromogenic agent D may be fixed in solid state on the reagent fixing part 205. Specifically, the second composition comprising glucitol reaction reagent R2 and/or the third composition comprising chromogenic agent D may be fixed in a dried state by being dispensed on at least one reagent fixing part 205.

According to one embodiment, the second composition comprising glucitol reaction reagent R2 (and/or the third composition comprising chromogenic agent D) may further include a stabilizer for fixing glucitol reaction reagent R2 in solid state on the reagent fixing part 205.

According to one embodiment, the stabilizer may be selected from the group consisting of sucrose, trehalose, lactose, maltose, turanose, cellobiose, dextran, diethylamino ethyl-dextran, dextrin, cellulose, and beta-glucans. According to a preferred embodiment, the stabilizer may be sucrose. Furthermore, the sucrose stabilizer may be added to the second composition at a concentration within a range of 90 mM to 360 mM. More preferably, sucrose may be added to the second composition at a concentration within a range of 160 mM to 200 mM. Most preferably, sucrose may be added to the second composition at a concentration of about 180 mM.

According to a preferred embodiment of the present application, the kit for quantitative analysis of 1,5-AG, method for quantitative analysis of 1,5-AG, and/or device for quantitative analysis of 1,5-AG can provide an effect of protecting the reaction enzymes from heat while allowing the reaction enzymes to sufficiently dissolve again for enzyme reaction by using sucrose at a concentration within a range of 90 mM to 360 mM as a stabilizer for glucitol reaction reagent R2. Furthermore, according to a preferred embodiment of the present application, the kit for quantitative analysis of 1,5-AG, method for quantitative analysis of 1,5-AG, and/or device for quantitative analysis of 1,5-AG can provide an effect of maintaining relatively high activity of the reaction enzymes even while storing for a relatively long period by fixing and storing the composition comprising glucitol reaction reagent R2, in solid state on the reagent fixing part.

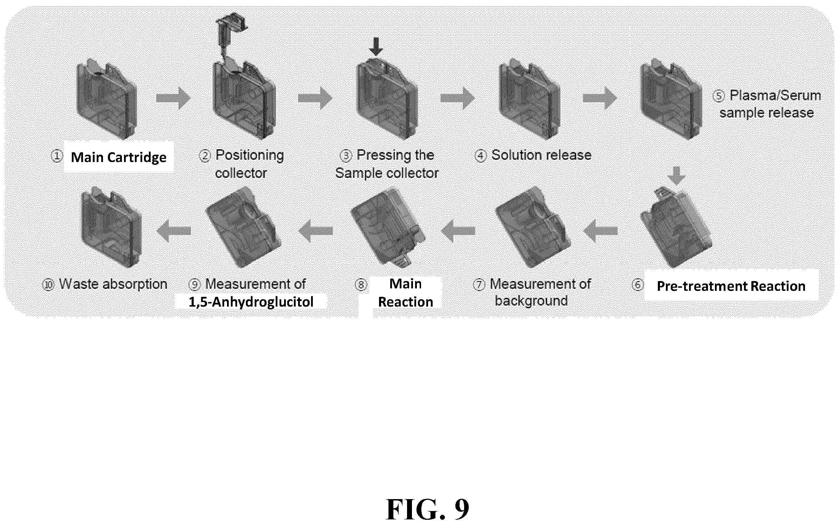

FIG. 9 is a diagram illustrating the analysis aspect for quantifying 1,5-AG in a sample according to an embodiment of the present application.

Referring to FIG. 9, the 1,5-AG quantitative analysis method according to an embodiment of the present application may be implemented as follows:
1) Prepare the main cartridge 200.
2) Position the sample collector 100 on the receiving part 201 of the main cartridge 200.
3) Apply pressure to the sample collector 100 in the direction toward the receiving part 201 of the main cartridge 200.
4) As the protrusion 102 of the sample collector 100 applies pressure to the solution cell 301, the solution cell 301 moves, as described above, through the moving frame 203 in the direction of the cover tape rupturing part 202. The cover tape rupturing part 202 comes into contact with the cover tape 302 of the solution cell 301, causing the solution (a solution containing the first composition) stored inside the solution cell 301 to flow into the mixing part 204 of the main cartridge 200.
5) As the solution flows into the mixing part 204, it comes into contact with the sample from the sample collector 100, causing the sample to also flow into the mixing part 204 of the main cartridge 200.
6) At this time, on the mixing part 204, the reaction (Pre-treatment Reaction in FIG. 9) between the solution containing glucose removal reagent R1 and the sample containing glucose proceeds, and at least a portion of glucose included in the sample is removed by the glucose removal reagent R1.
   Meanwhile, according to one embodiment of the present application, when external force (for example, rotational force applied by the quantitative analysis device to be described later) is applied to the kit 10 for quantitative analysis of 1,5-AG, the kit 10 for quantitative analysis of 1,5-AG rotates by a predetermined angle, and accordingly, the sample in the main cartridge 200 can move through the flow path 206 of the main cartridge 200 by gravity.
7) By external force applied to the kit 10 for quantitative analysis of 1,5-AG, the sample from which at least a portion of glucose has been removed moves to the measurement unit 207, and the background absorbance may be measured while the sample is positioned on the measurement unit 207.
8) By external force applied to the kit 10 for quantitative analysis of 1,5-AG, the sample from which at least a portion of glucose has been removed moves to the reagent fixing part 205 where glucitol reaction reagent R2 and/or chromogenic agent D are fixed, and enzyme reaction (Main Reaction in FIG. 9) between 1,5-AG included in the sample and glucitol reaction reagent R2 proceeds on the reagent fixing part 205. Hydrogen peroxide is generated through the enzyme reaction, and the chromogenic agent D is reduced and changes color due to electrons released by oxidizing the generated hydrogen peroxide.
9) By external force applied to the kit 10 for quantitative analysis of 1,5-AG, the sample that has undergone enzyme reaction moves to the measurement unit 207, and final absorbance may be measured while the sample is positioned on the measurement unit 207. Furthermore, 1,5-AG may be quantified based on the background absorbance and final absorbance.
10) The waste from the completed quantitative analysis moves from the measurement unit 207 to the waste solution treatment part 208 due to the external force applied to the 1,5-AG quantitative analysis kit 10, where it is collected.

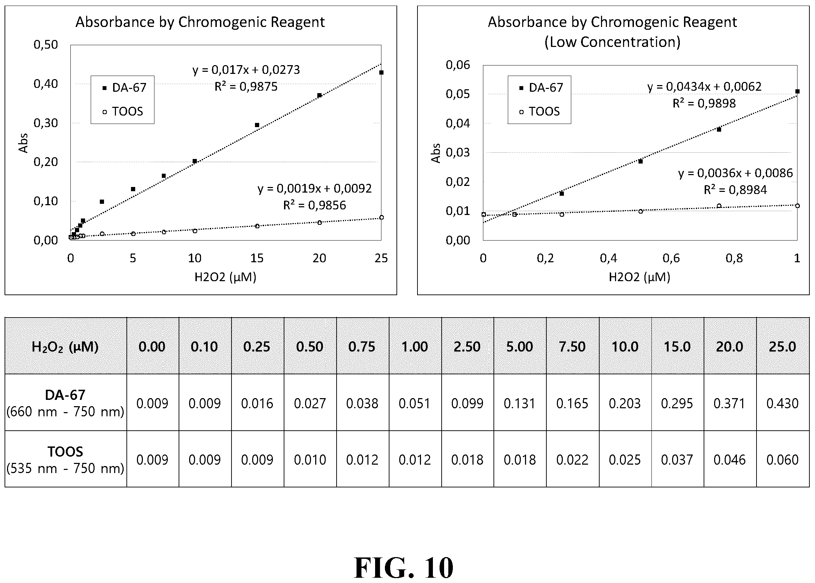

Hereinafter, with reference to FIGS. 10 to 15, the present invention will be described in detail through experimental examples. However, the following experimental examples are merely illustrative and should not be construed as limiting.

### <Experimental Example 1: Evaluation of Absorbance by Chromogenic Agent>

According to a preferred embodiment of the present application, as described above, the chromogenic agent oxidized from hydrogen peroxide generated by enzyme reaction may be DA-67.

### 1. Experimental Method

To evaluate the performance of reagents for quantitative analysis of 1,5-AG, absorbance according to hydrogen peroxide concentration was measured for DA-67 chromogenic agent used in this invention and TOOS chromogenic agent widely used conventionally. Specifically, absorbance of DA-67 chromogenic agent and TOOS chromogenic agent was measured according to hydrogen peroxide concentration on a 96-well plate. Additionally, absorbance was measured while varying hydrogen peroxide concentration at 0, 0.1, 0.25, 0.50, 0.75, 1.00, 2.50, 5.00, 7.50, 10.0, 15.0, 20.0, 25.0 µM.

The types and concentrations of reagents used were as follows:
- DA-67 (Sodium 10-(carboxymethylaminocarbonyl)-3,7-bis(dimethylamino)phenothiazine, Wako), 12.7 µM
- TOOS (3-(N-Ethyl-3-methylanilino)-2-hydroxypropanesulfonic acid sodium salt, Sigma), 12.7 µM
   4-AAP (4-Aminoantipyrine, Sigma), 12.7 µM (coupler reagent)
- POD (Peroxidase, TOYOBO), 34.5 U/mL
- H2O2 (Hydrogen Peroxide, Sigma)

DA-67 absorbance was measured using Thermo Scientific's Multiskan Go 1510 UV/VIS Spectrophotometer, specifically calculated by subtracting absorbance measured at 750 nm wavelength from absorbance measured at 660 nm wavelength.

TOOS absorbance was measured using Thermo Scientific's Multiskan Go 1510 UV/VIS Spectrophotometer, specifically calculated by subtracting absorbance measured at 750 nm wavelength from absorbance measured at 535 nm wavelength.

### 2. Experimental Results

FIG. 10 is a graph and table showing evaluation results of absorbance by chromogenic agent according to one embodiment of the present application.

Referring to FIG. 10, compared to TOOS chromogenic agent at the same concentration, DA-67 chromogenic agent was observed to have relatively higher absorbance for substantially all hydrogen peroxide concentrations. Particularly, considering that DA-67 chromogenic agent (slope value: 0.0434) showed relatively higher slope value compared to TOOS chromogenic agent (slope value: 0.0036) for hydrogen peroxide in low concentration range (1 µM or less), it can be confirmed that DA-67 chromogenic agent is relatively superior in ability to differentiate hydrogen peroxide in low concentration ranges.

### <Experimental Example 2: Evaluation of Stability According to State of Reaction Reagent>

According to a preferred embodiment of the present application, as described above, the composition containing glucitol reaction reagent R2 may be fixed in solid state on the reagent fixing part.

### 1. Experimental Method

To compare stability according to state of the reaction enzymes used for quantitative analysis of 1,5-AG, absorbance was comparatively analyzed between cases where reaction enzymes were stored in solid state and cases where reaction enzymes were stored in liquid state. Specifically, absorbance was measured according to storage days while storing reaction enzymes in solid state in a constant temperature and humidity room at room temperature, and absorbance was measured according to storage days while storing reaction enzymes in liquid state in a constant temperature and humidity room at room temperature. Additionally, absorbance was measured while varying 1,5-AG concentration at 0 µg/mL, 6 µg/mL, and 20 µg/mL. As described above, DA-67 absorbance was measured using i-SENS's A1Care Analyzer, specifically calculated by subtracting absorbance measured at 750 nm wavelength from absorbance measured at 660 nm wavelength.

The types and concentrations of reagents used were as follows:
- DA-67 (Sodium 10-(carboxymethylaminocarbonyl)-3,7-bis(dimethylamino)phenothiazine, Wako), 3.0 µg/assay
- PROD (Pyranose Oxidase from Microorganism, Creative Enzyme), 7.5 U/assay
- POD (Peroxidase, TOYOBO), 12.0 U/assay
- 1,5-AG (1,5-Anhydroglucitol, Sigma)
- Sucrose (180 mM)

Meanwhile, stability was evaluated by adding the same amount of sucrose to liquid samples as solid samples.

### 2. Experimental Results

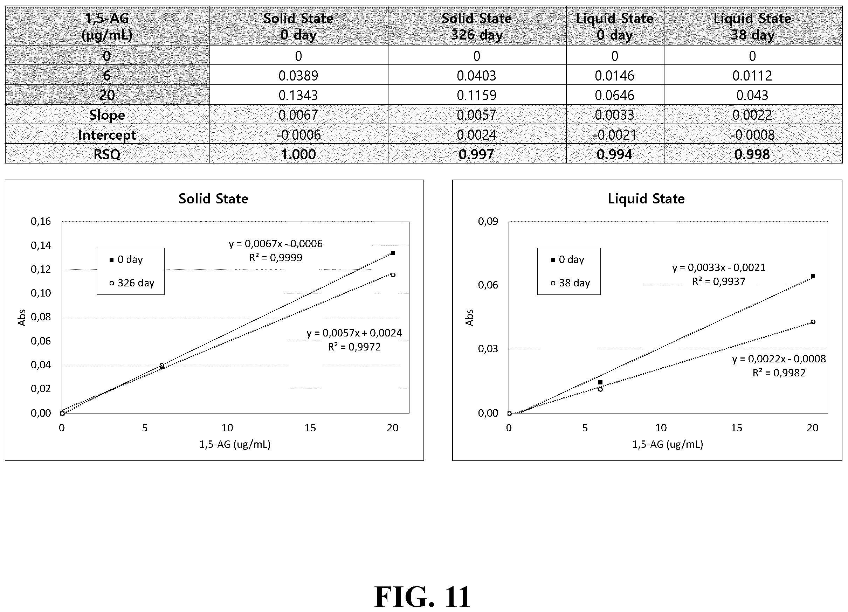

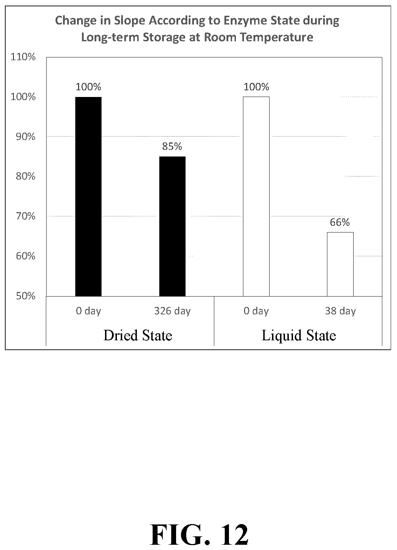

FIG. 11 is a table and graph showing evaluation results of stability according to state of the glucitol reaction reagent according to one embodiment of the present application. FIG. 12 is a graph showing evaluation results of stability according to state of the glucitol reaction reagent according to one embodiment of the present application.

Referring to FIGS. 11 and 12, when reaction enzymes (PROD, POD) were stored in solid state, the slope of absorbance by 1,5-AG concentration was observed to be 0.0067 at the start of storage and 0.0057 after 326 days from the start of storage. Meanwhile, when reaction enzymes (PROD, POD) were stored in liquid state, the slope of absorbance by 1,5-AG concentration was observed to be 0.0033 at the start of storage and 0.0022 after 38 days from the start of storage. Here, the slope indicates the activity of reaction enzymes.

In other words, when the composition containing glucitol reaction reagent R2 was fixed and stored in solid state on the reagent fixing part, it was confirmed that the activity of glucitol reaction reagent (for example, PROD and POD) was maintained at 85% of initial activity even after 326 days from the start of storage. Meanwhile, when the composition containing glucitol reaction reagent R2 was implemented in liquid state, it was confirmed that the activity of glucitol reaction reagent (for example, PROD and POD) was maintained at only about 66% of initial activity after just 38 days from the start of storage. Through this experimental example, it was confirmed that storing reaction enzymes in solid state has significantly superior stability compared to storing in liquid state and is also advantageous for reaction enzyme activity. Furthermore, it was additionally confirmed that storing in solid state showed relatively higher activity of reaction enzymes compared to storing in liquid state for all storage periods.

According to one embodiment of the present application, by fixing and storing the composition containing glucitol reaction reagent in solid state on the reagent fixing part, the activity of the glucitol reaction reagent is maintained at 66% or more of its initial activity, preferably at 80% or more, even after 326 days from the start of storage.

According to one embodiment of the present application, by fixing and storing the composition containing glucitol reaction reagent in solid state on the reagent fixing part, the activity of the glucitol reaction reagent is maintained at 66% or more of its initial activity, preferably at 85% or more, even after 38 days from the start of storage.

### <Experimental Example 3: Evaluation of Absorbance According to Type of Stabilizer>

According to a preferred embodiment of the present application, as described above, the stabilizer for storing glucitol reaction reagent R2 in solid state may be sucrose.

### 1. Experimental Method

To evaluate performance by stabilizer of glucitol reaction reagent R2 used for quantitative analysis of 1,5-AG, background absorbance was primarily measured between cases where trehalose was added as a stabilizer to glucitol reaction reagent R2 and cases where trehalose was not added as a stabilizer to glucitol reaction reagent R2. Additionally, background absorbance was comparatively analyzed between cases where trehalose was added as a stabilizer to glucitol reaction reagent R2 and cases where sucrose was added as a stabilizer to glucitol reaction reagent R2.

The types and concentrations of reagents used were as follows:
- DA-67 (Sodium 10-(carboxymethylaminocarbonyl)-3,7-bis(dimethylamino)phenothiazine, Wako), 3.0 µg/assay
- PROD (Pyranose Oxidase from Microorganism, Creative Enzyme), 7.5 U/assay
- POD (Peroxidase, TOYOBO), 12.0 U/assay
- 1,5-AG (1,5-Anhydroglucitol, Sigma)
- Stabilizer
   1) Trehalose (0mM), 2) Trehalose (180mM), 3) Sucrose (180 mM)

### 2. Experimental Results

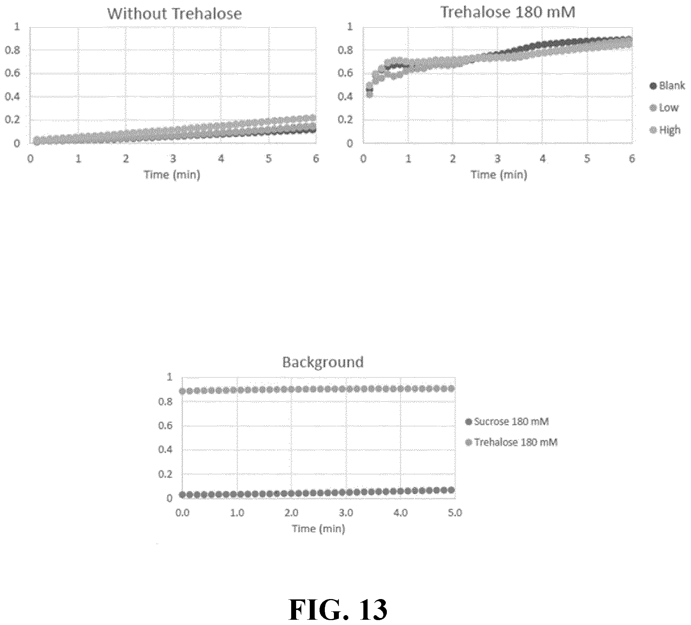

FIG. 13 is a graph showing evaluation results of absorbance according to stabilizer added to the glucitol reaction reagent according to one embodiment of the present application.

Referring to FIG. 13, when using trehalose as a stabilizer, background absorbance increased compared to when not using trehalose as a stabilizer, and concentration-dependent tendency disappeared. This result is predicted to be derived from glucitol reaction reagent (PROD) additionally recognizing trehalose besides 1,5-AG Meanwhile, when using sucrose as a stabilizer, it was confirmed that background absorbance did not substantially increase, unlike when using trehalose as a stabilizer.

Through this experimental example, it was confirmed that sucrose is preferable as the stabilizer added to glucitol reaction reagent R2 for quantitative analysis of 1,5-AG, because it shows substantially weak background absorbance, which means it does not affect the quantitative analysis of 1,5-AG Therefore, according to a preferred embodiment of the present application, the stabilizer for storing glucitol reaction reagent R2 in solid state may be sucrose. However, sucrose is merely exemplary, and any appropriate disaccharide (for example, lactose, maltose, turanose, cellobiose) or any appropriate polysaccharide (dextran, diethylamino ethyl-dextran, dextrin, cellulose, and beta-glucans) may be adopted as a stabilizer for achieving the purpose of storing glucitol reaction reagent in solid state while not affecting quantitative analysis of 1,5-AG

According to one embodiment of the present application, the stabilizer added to glucitol reaction reagent R2 may be sucrose, and sucrose may be added to glucitol reaction reagent R2 at a concentration within a range of 90 mM to 360 mM.

### <Experimental Example 4: Evaluation of Absorbance According to Concentration of Stabilizer (Sucrose)>

### 1. Experimental Method

To evaluate the optimal content of stabilizer (Sucrose) used for drying glucitol reaction reagent R2 for quantitative analysis of 1,5-AG, absorbance was measured according to 1,5-AG concentration while varying sucrose content. More specifically, sucrose was added to glucitol reaction reagent (PROD, POD) at contents of 90 mM, 180 mM, and 360 mM respectively. Furthermore, absorbance was measured for each sample while varying 1,5-AG concentration at 0 µg/mL, 6 µg/mL, 14 µg/mL, and 20 µg/mL.

As described above, absorbance was measured using i-SENS's A1Care Analyzer, specifically calculated by subtracting absorbance measured at 750 nm wavelength from absorbance measured at 660 nm wavelength. Furthermore, absorbance indicating activity of glucitol reaction reagent (PROD, POD) was measured based on absorbance at 5 min and absorbance at 0 min.

The types and concentrations of reagents used were as follows:
- DA-67 (Sodium 10-(carboxymethylaminocarbonyl)-3,7-bis(dimethylamino)phenothiazine, Wako), 3.0 µg/assay
- PROD (Pyranose Oxidase from Microorganism, Creative Enzyme), 7.5 U/assay
- POD (Peroxidase, TOYOBO), 12.0 U/assay
- Sucrose
   1) 90 mM, 2) 180 mM, 3) 360 mM
- 1,5-AG (1,5-Anhydroglucitol, Sigma)

### 2. Experimental Results

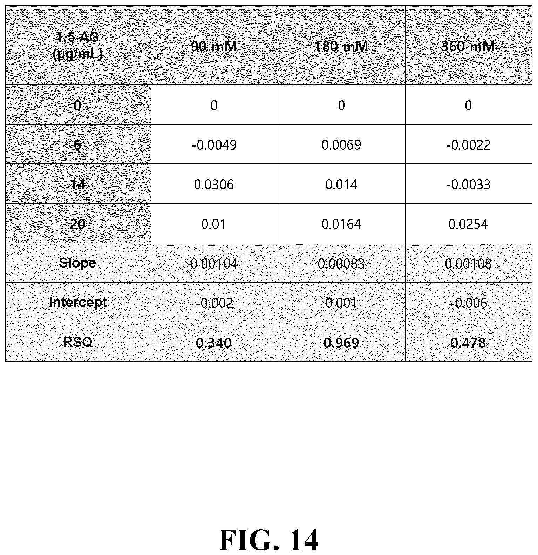

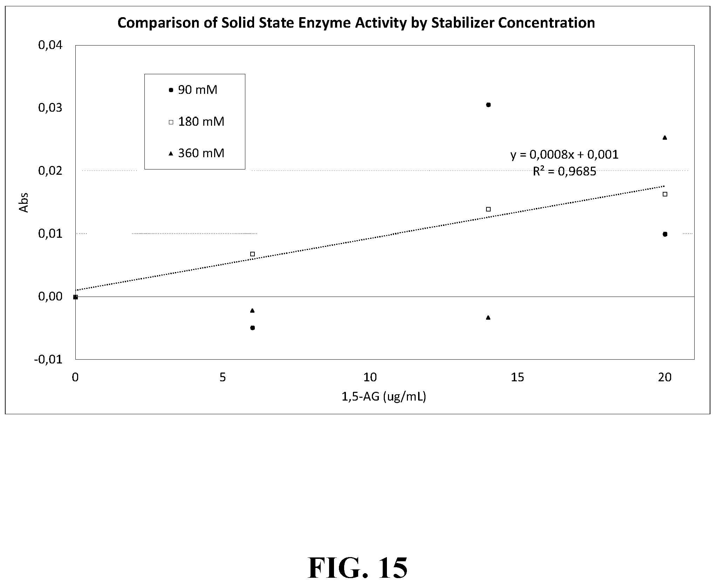

FIG. 14 is a table showing evaluation results of absorbance according to concentration of stabilizer added to the glucitol reaction reagent according to one embodiment of the present application. FIG. 15 is a graph showing evaluation results of absorbance according to concentration of stabilizer added to the glucitol reaction reagent according to one embodiment of the present application.

Referring to FIGS. 14 and 15, when sucrose was added as a stabilizer to glucitol reaction reagent at contents of 90 mM and 360 mM, absorbance showed no tendency. That is, it was confirmed that when sucrose was added as a stabilizer to glucitol reaction reagent at contents of 90 mM and 360 mM, unstable enzyme activity was shown. Furthermore, when sucrose was added as a stabilizer to glucitol reaction reagent at a content of 360 mM, it was observed that reaction enzymes (PROD, POD) of the dried solid glucitol reaction reagent did not dissolve again due to excessive addition of stabilizer. Through this experimental example, it was confirmed that when adding sucrose stabilizer at a concentration within a range of 90 mM to 360 mM, preferably at 180 mM, reaction enzymes for quantitative analysis of 1,5-AG showed optimal activity.

Therefore, according to one embodiment of the present application, the sucrose stabilizer may be added to the composition containing glucitol reaction reagent at a concentration within a range of 90 mM to 360 mM. According to a preferred embodiment, the sucrose stabilizer may be added to the composition containing glucitol reaction reagent at a concentration within a range of 160 mM to 200 mM. More preferably, the sucrose stabilizer may be added to the composition containing glucitol reaction reagent at a concentration of about 180 mM.

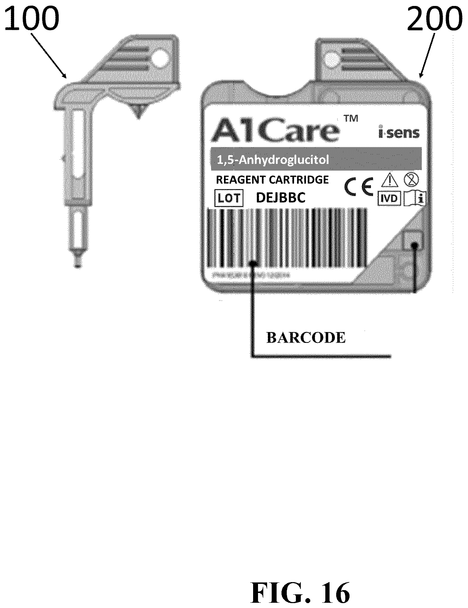

FIG. 16 is a diagram showing identification information included in the 1,5-AG quantitative analysis kit according to an embodiment of the present application.

The 1,5-AG quantitative analysis kit 10 according to one embodiment of the present application may include identification information for recognizing a biological sample. For example, the 1,5-AG quantitative analysis kit 10 may include identification information (e.g., information in the form of a barcode) on one surface of the main cartridge 200 to identify the type of biological sample.

The quantitative analysis apparatus(or quantitative analysis device), described later, may acquire identification information to recognize the type of biological sample to be analyzed by the 1,5-AG quantitative analysis kit 10. For instance, the quantitative analysis device may determine, based on a barcode, that the biological sample for analysis is 1,5-AG In this case, the quantitative analysis device may be configured to perform quantitative analysis of the biological sample by executing a pre-stored analysis protocol associated with the recognized type, based on the recognized type of the biological sample. For example, the quantitative analysis device may be implemented to execute a pre-stored 1,5-AG analysis protocol based on recognizing that the type of biological sample to be analyzed is 1,5-AG, thereby performing quantitative analysis of 1,5-AG

Meanwhile, FIG. 16 illustrates identification information in the form of a barcode as an example. However, this is merely an example, and any suitable form of identification information may be provided at any suitable location on the 1,5-AG quantitative analysis kit 10.

The quantitative analysis device according to an embodiment of the present disclosure may be configured to perform quantitative analysis of biological samples (e.g., 1,5-AG, glycated albumin, CRP, etc.). Furthermore, the quantitative analysis device may include a communication module (which may also be referred to as a transceiver), a memory, and/or a processor.

The communication module of the quantitative analysis device may communicate with any external device or external server. For example, the quantitative analysis device may transmit quantitative analysis results to an external device or external server via the communication module.

The quantitative analysis device may access a network through the communication module to transmit or receive various types of data. The communication module may largely include a wired-type communication module and a wireless-type communication module. Since the wired-type communication module and the wireless-type communication module have their own advantages and disadvantages, in some cases, the wired-type communication module and the wireless-type communication module may be provided together as the quantitative analysis device. Here, in the case of the wireless-type communication module, a wireless local area network (WLAN) type communication method such as Wi-Fi may be mainly used. Alternatively, in the case of the wireless-type communication module, cellular communication, such as long-term evolution (LTE) or 5G communication methods, may be used. However, a wireless communication protocol is not limited to the above-described example, and any appropriate wireless type of communication method may be used. In the case of the wired-type communication module, a local area network (LAN) or Universal Serial Bus (USB) communication is a representative example, but other methods may be used.

Various types of information may be stored in the memory of the quantitative analysis device. Various types of data may be temporarily or semi-permanently stored in the memory. Examples of the memory may include a hard disk drive (HDD), a solid state drive (SSD), a flash memory, a read-only memory (ROM), a random access memory (RAM), etc. The memory may be provided to be embedded in the e quantitative analysis device or in a detachable form. Various types of data necessary for the operation of the quantitative analysis device, including an operating program (OS) for driving the quantitative analysis device or a program for operating each component of the quantitative analysis device, may be stored in the memory.

The processor may control the overall operation of the quantitative analysis device. For example, the quantitative analysis device may control its overall operations, including actions such as recognizing the identification information of a biological sample, executing a corresponding analysis protocol based on the recognized identification information, and/or performing quantitative analysis of the biological sample according to the analysis protocol. Specifically, the processor may load and execute a program for the overall operation of the quantitative analysis device from the memory. The processor may be implemented as an application processor (AP), a central processing unit (CPU), a microcontroller unit (MCU), or a similar device depending on hardware, software, or a combination thereof. In this case, the processor may be provided in the form of an electronic circuit that processes electrical signals and performs a control function in hardware, and may be provided in the form of a program or code that drives a hardware circuit in software.

According to one embodiment of the present invention, a kit for quantitative analysis of 1,5-anhydroglucitol, a method for quantitative analysis of 1,5-anhydroglucitol, and/or a device for performing the same can provide a miniaturization effect by using a single DA-67 chromogenic agent.

According to one embodiment of the present invention, a kit for quantitative analysis of 1,5-anhydroglucitol, a method for quantitative analysis of 1,5-anhydroglucitol, and/or a device for performing the same can provide enhanced analytical capability of 1,5-AG quantitative analysis by more precisely differentiating hydrogen peroxide in low concentration ranges using DA-67 chromogenic agent.

According to one embodiment of the present invention, a kit for quantitative analysis of 1,5-anhydroglucitol, a method for quantitative analysis of 1,5-anhydroglucitol, and/or a device for performing the same can provide an effect of protecting the reaction reagent from heat while allowing the reaction reagent to sufficiently dissolve again for enzyme reaction by using sucrose at optimal concentration as a stabilizer for the reaction reagent.

According to one embodiment of the present invention, a kit for quantitative analysis of 1,5-anhydroglucitol, a method for quantitative analysis of 1,5-anhydroglucitol, and/or a device for performing the same can provide an effect of maintaining high activity of the reaction reagent while storing for a relatively long period by implementing the reaction reagent in solid state.

Effects of the present invention are not limited to the above-described effects and other effects that are not described may be clearly understood by those skilled in the art from the above detailed descriptions.

Features, structures, and effects described in the above-described exemplary embodiments are included in at least one exemplary embodiment of the present invention, but are not necessarily limited to only one exemplary embodiment. Furthermore, features, structures, and effects described in each embodiment can be combined or modified and implemented in other embodiments by one of ordinary skill in the art to which the embodiments belong. Therefore, it should be interpreted that contents related to such combinations and modifications are included in the scope of the present invention.

Further, while the present invention has been particularly described with reference to embodiments, the embodiments are only exemplary embodiments of the present invention and the present invention is not intended to be limited thereto. It will be understood by those skilled in the art that modifications and applications in other forms may be made without departing from the spirit and scope of the present invention. That is, each element specifically shown in the embodiments may be modified and embodied. In addition, it should be understood that differences related to these modifications and applications are within the scope of the present invention as defined in the appended claims.

## Claims

1. A kit for quantitative analysis of 1,5-AG comprising:
a first composition comprising a glucose removal reagent for removing at least a portion of glucose in a sample;
a second composition comprising a glucitol reaction reagent for enzyme reaction of 1,5-anhydroglucitol included in the sample from which at least a portion of glucose has been removed; and
a third composition comprising a chromogenic agent being oxidized from hydrogen peroxide (H2O2) generated by enzyme reaction of the 1,5-anhydroglucitol,
wherein the kit for quantitative analysis of 1,5-AG comprises at least one reagent fixing part,
wherein the second composition and the third composition are independently fixed in solid state to the reagent fixing part in respectively,
wherein the second composition further comprises a stabilizer for fixing the glucitol reaction reagent in solid state to the reagent fixing part, and
wherein the chromogenic agent is DA-67.

2. The kit for quantitative analysis of 1,5-AG according to claim 1, wherein the stabilizer is selected from the group consisting of sucrose, trehalose, lactose, maltose, turanose, cellobiose, dextran, diethylamino ethyl-dextran, dextrin, cellulose, and beta-glucans.

3. The kit for quantitative analysis of 1,5-AG according to claim 2,
wherein the stabilizer is sucrose, and
wherein the sucrose is added to the second composition at a concentration within a range of 90 mM to 360 mM.

4. The kit for quantitative analysis of 1,5-AG according to claim 2,
wherein the stabilizer is sucrose, and
wherein the sucrose is added to the second composition at a concentration within a range of 160 mM to 200 mM.

5. The kit for quantitative analysis of 1,5-AG according to claim 1, wherein the glucose removal reagent comprises:
a glucose converting enzyme for generating a product including glucose-6-phosphate from a reactant including the glucose in the sample; and
a reverse reaction removal reagent for inhibiting a reverse reaction from the glucose-6-phosphate back to the glucose.

6. The kit for quantitative analysis of 1,5-AG according to claim 5, wherein the glucose converting enzyme is selected from the group consisting of glucokinase (GK), hexokinase, and mixtures thereof.

7. The kit for quantitative analysis of 1,5-AG according to claim 6, wherein the reverse reaction removal reagent comprises pyruvate kinase and phosphoenolpyruvic acid (PEP).

8. The kit for quantitative analysis of 1,5-AG according to claim 7, wherein the reactant further comprises ATP.

9. The kit for quantitative analysis of 1,5-AG according to claim 1, wherein the glucitol reaction reagent comprises:
a first reaction enzyme generating hydrogen peroxide by reacting with the 1,5-AG in the sample; and
a second reaction enzyme oxidizing the chromogenic agent by reacting with the hydrogen peroxide.

10. The kit for quantitative analysis of 1,5-AG according to claim 9, wherein the first reaction enzyme is pyranose oxidase (PROD).

11. The kit for quantitative analysis of 1,5-AG according to claim 10, wherein the second reaction enzyme is peroxidase (POD).

12. The kit for quantitative analysis of 1,5-AG according to claim 1, wherein by fixing and storing the second composition comprising the glucitol reaction reagent in solid state to the reagent fixing part, the activity of the glucitol reaction reagent is maintained at 66% or more of its initial activity even after 38 days from the start of storage.

13. The kit for quantitative analysis of 1,5-AG according to claim 1, wherein by fixing and storing the second composition comprising the glucitol reaction reagent in solid state to the reagent fixing part, the activity of the glucitol reaction reagent is maintained at 85% or more of its initial activity even after 38 days from the start of storage.

14. The kit for quantitative analysis of 1,5-AG according to claim 1, wherein by fixing and storing the second composition comprising the glucitol reaction reagent in solid state to the reagent fixing part, the activity of the glucitol reaction reagent is maintained at 66% or more of its initial activity even after 326 days from the start of storage.

15. The kit for quantitative analysis of 1,5-AG according to claim 1, wherein by fixing and storing the second composition comprising the glucitol reaction reagent in solid state to the reagent fixing part, the activity of the glucitol reaction reagent is maintained at 80% or more of its initial activity even after 326 days from the start of storage.

16. The kit for quantitative analysis of 1,5-AG according to claim 1,
wherein the kit comprises a body including an upper plate and a lower plate configured to face each other,
wherein the second composition is fixed to a first reagent fixing part located in a first compartmental region of the upper plate,
wherein the third composition is fixed to a second reagent fixing part located in the first compartmental region of the lower plate, and
wherein the first reagent fixing part and the second reagent fixing part are configured to face each other.

17. The kit for quantitative analysis of 1,5-AG according to claim 16,
wherein the kit comprises a solution cell disposed in a second compartmental region partitioned from the first compartmental region,
wherein the first composition is stored in the solution cell,
wherein when a sample collector containing the sample is inserted into a receiving part of the kit, the first composition contained in the solution cell and the sample contained in the sample collector are configured to flow into a mixing region within the second compartmental region such that at least a portion of glucose in the sample is removed in the mixing region.

18. The kit for quantitative analysis of 1,5-AG according to claim 17,
wherein the kit further comprises a flow path connecting the first compartmental region and the second compartmental region,
wherein the sample from which at least a portion of glucose has been removed flows from the mixing region within the second compartmental region to the first compartmental region through the flow path as the kit is rotated by a predetermined angle by external force applied to the kit.
